# EUROPEAN PATENT APPLICATION

(11) **EP 4 335 449 A1**
(43) Date of publication of application: **13.03.2024**
(21) Application number: 23193883.8
(22) Date of filing: 29.08.2023
(51) Int. Cl.: A61K 36/062, A61K 36/258, A61K 36/43, A61P 25/20, A61P 25/24, A61P 25/28

(54) **COMPOSITION OF BOTANICAL EXTRACTS FOR SLEEP AND MOOD SUPPORT**

(30) Priority: 29.08.2022 US 202263401876 P
(71) Applicant: NuLiv Science USA, Inc., Brea, CA 92821 (US)
(72) Inventor: Wang, Michael Chang Yu, Brea, 92821 (US)
(74) Representative: Straus, Alexander

(57) **Abstract**

The present invention provides a composition for sleep and mood support, which comprises composition for sleep and mood support, which consists essentially of *Xylaria nigripes* extract, *Panax notoginseng* extract and *Cuscuta chinensis* extract, as main active components at a relative ratio to provide a synergistic effect in increasing GABA content, and GABA-related enzyme activities, and decreasing β-amyloid protein aggregation. Also provided is the use of the composition for manufacturing a formulation for treating insomnia, depression and/or various neurological conditions.

## Description

### FILED OF THE INVENTION

The invention concerns a composition of botanical extracts for sleep and mood support, which provides synergistic effects in improving sleep quality, promoting relaxation, and elevating mood.

### BACKGROUND Z OF THE INVENTION

γ-Aminobutyric acid (GABA) is a non-proteinogenic amino acid that acts as an inhibitory neurotransmitter, which helps to block the neuro signals/message and thereby reduce stress, anxiety, and fear (calming effect). Hence GABA plays a crucial role in stabilizing mood (Sarasa et al., 2020). Researchers are showing immense interest in GABA and its uptake to regulate various neuropsychological issues, which are related to hyper neuronal excitability and thus useful for treating autism, schizophrenia, Tourette's syndrome, and other hyperactive neuronal disorders (Sarawagi et al., 2021; Kendell et al., 2005). GABA is produced from glutamate/glutamic acid (in GABA-ergic neuron) with the help of GABA decarboxylase (GAD) enzyme and degraded/broken down by GABA transaminase or aminotransferase (GABA-T) enzyme therefore to maintain a nominal level of GABA in the system, the above-mentioned enzymes play a pivotal role (Ngo et al., 2019). Moreover, studies have shown that GABA has a strong association with insomnia, hypertension, and Alzheimer's disease as it balances the production of various neurotransmitters/neurotropic factors and hormones (Yu et al., 2020; Solas et al., 2015). Recently, many scientists have shown interest in regulating GABA levels (nominal level) to maintain a strong healthy mental status in normal and various neurological disorders subjects using various traditional medicine, which has traditionally been used in regulating mood, sleep, and stress/depression (Yu et al., 2020; Byun et al., 2018).

It is still desirable to develop a new composition for sleep and mood support based on the study on GABA.

### SUMMARY OF THE INVENTION

Accordingly, it is unexpectedly found in the invention that a composition consisting of three botanical extracts/ingredients exhibits a synergistic effect in increasing GABA content, and GABA-related enzyme activities, and decreasing β-amyloid protein aggregation in PC 12 cells, so as to be a potent candidate for treating insomnia, depression and various neurological conditions.

In one aspect, the invention provides a composition for sleep and mood support, which consists essentially of comprises *Xylaria nigripes* extract, *Panax notoginseng* extract and *Cuscuta chinensis* extract at a relative ratio to provide a synergistic effect in increasing GABA content, and GABA-related enzyme activities, and decreasing β-amyloid protein aggregation.

In one embodiment of the invention, the composition consists of *Xylaria nigripes* extract, *Panax notoginseng* extract and *Cuscuta chinensis* extract, as main active components, at a relative ratio to provide a synergistic effect in increasing GABA content, and GABA-related enzyme activities, and decreasing β-amyloid protein aggregation, and optionally an edible carrier.

In one further embodiment of the invention, the composition consists of 50%-96% fermented *Xylaria nigripes* extract, 0.001%-10% *Panax notoginseng* extract and 4%-50% *Cuscuta chinensis* extract, and optionally an edible carrier.

In one preferred embodiment of the invention, the composition consists of 70%-90% *Xylaria nigripes* extract, 0.01%-5% *Panax notoginseng* extract and 10%-30% *Cuscuta chinensis* extract, and optionally an edible carrier.

In one more preferred embodiment of the invention, the composition consist of 80%-90% fermented *Xylaria nigripes* extract, 0.05%-1% *Panax notoginseng* extract and 10%-20% *Cuscuta chinensis* extract, and optionally an edible carrier.

In another aspect, the present invention provides a method for treating insomnia, depression or a neurological condition in a subject, comprising administering the subject the composition according to the invention.

It is to be understood that both the foregoing general description and the following detailed description are exemplary and explanatory only and are not restrictive of the invention.

### BRIEF DISCRIPTION OF THE DRAWINGS

The foregoing description and the following detailed description of the invention will be better understood when reading in conjunction with the accompanying drawings. For the purpose of illustrating the present invention, currently preferred embodiments are shown in the drawings.

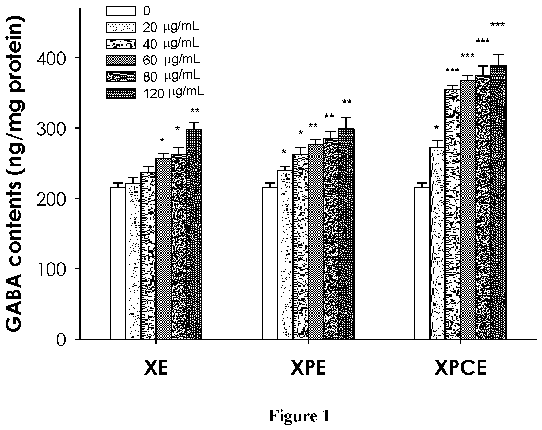

Figure 1 shows the impact of the three compositions on GABA content in PC12 cells: XE *(Xylaria nigripes* extract), XPE *(Xylaria nigripes* extract and *Panax notoginseng* extract) and XPCE *(Xylaria nigripes* extract, *Panax notoginseng* extract and *Cuscuta chinensis* extract) according to the invention. All the experiments were performed in triplicate (n=3). Data are expressed as the mean ± standard deviation (SD). *p < 0.05, **p < 0.01, ***p < 0.001 vs control.

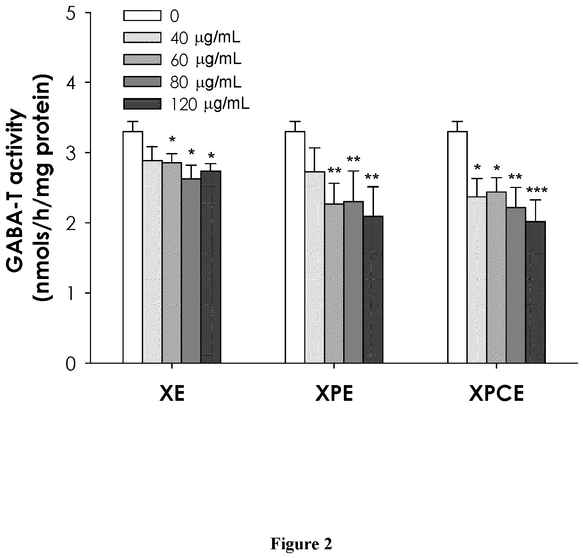

Figure 2 shows the impact of the three compositions on *GABA Transaminase (GABA-T) activity* in PC12 cells: XE *(Xylaria nigripes* extract), XPE *(Xylaria nigripes* extract and *Panax notoginseng* extract) and XPCE *(Xylaria nigripes* extract, *Panax notoginseng* extract and *Cuscuta chinensis* extract) according to the invention. All the experiments were performed in triplicate (n=3). Data are expressed as the mean ± standard deviation (SD). *p < 0.05, **p < 0.01, ***p < 0.001 vs control.

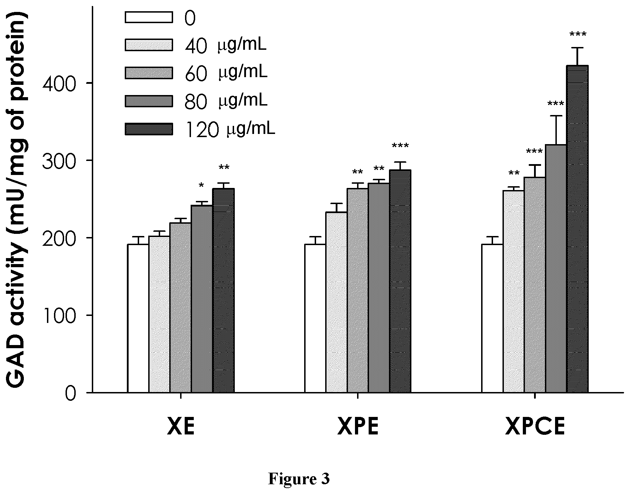

Figure 3 shows the impact of the three compositions on *GABA Decarboxylase (GAD) activity* in PC12 cells: XE *(Xylaria nigripes* extract), XPE *(Xylaria nigripes* extract and *Panax notoginseng* extract) and XPCE *(Xylaria nigripes* extract, *Panax notoginseng* extract and *Cuscuta chinensis* extract) according to the invention. All the experiments were performed in triplicate (n=3). Data are expressed as the mean ± standard deviation (SD). *p < 0.05, **p < 0.01, ***p < 0.001 vs control.

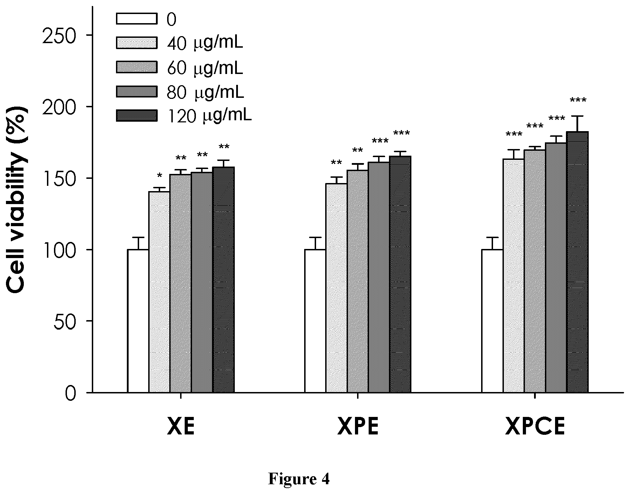

Figure 4 shows the impact of the three compositions on the cells viability without Aβ 1-42 peptide exposure in PC12 cells: XE *(Xylaria nigripes* extract), XPE *(Xylaria nigripes* extract and *Panax notoginseng* extract) and XPCE *(Xylaria nigripes* extract, *Panax notoginseng* extract and *Cuscuta chinensis* extract) according to the invention. All the experiments were performed in triplicate (n=3). Data are expressed as the mean ± standard deviation (SD). *p < 0.05, **p < 0.01, ***p < 0.001 vs control.

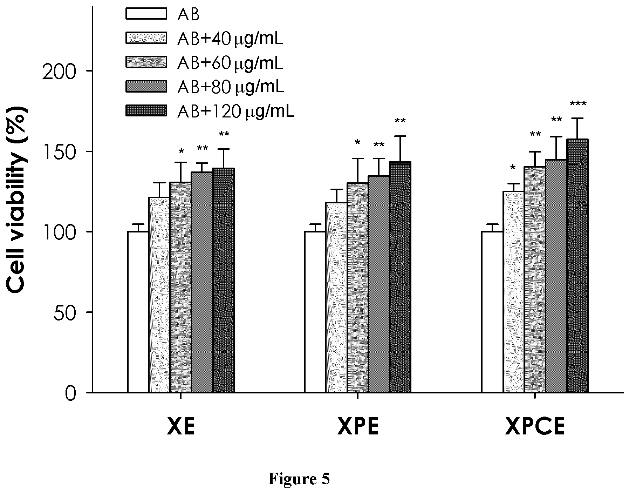

Figure 5 shows the impact of the three compositions on Cells viability with Aβ 1-42 peptide exposure in PC12 cells: XE *(Xylaria nigripes* extract), XPE (*Xylaria nigripes* extract and *Panax notoginseng* extract) and XPCE *(Xylaria nigripes* extract, *Panax notoginseng* extract and *Cuscuta chinensis* extract) according to the invention. All the experiments were performed in triplicate (n=3). Data are expressed as the mean ± standard deviation (SD). *p < 0.05, **p < 0.01, ***p < 0.001 vs control.

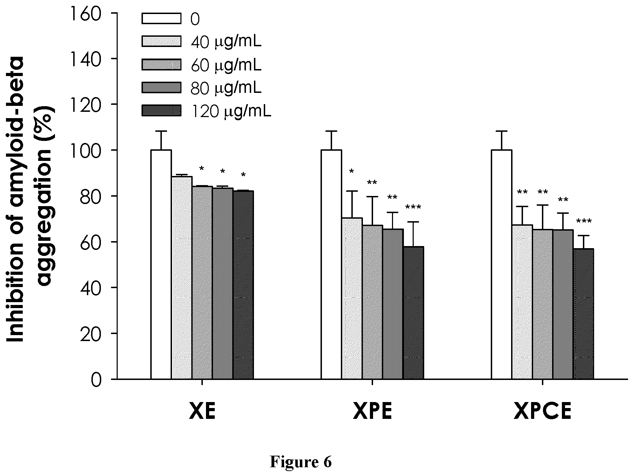

Figure 6 shows the impact of the three compositions on Aβ protein Aggregation in PC12 cells: XE *(Xylaria nigripes* extract), XPE *(Xylaria nigripes* extract and *Panax notoginseng* extract) and XPCE *(Xylaria nigripes* extract, *Panax notoginseng* extract and *Cuscuta chinensis* extract) according to the invention. All the experiments were performed in triplicate (n=3). Data are expressed as the mean ± standard deviation (SD). *p < 0.05, **p < 0.01, ***p < 0.001 vs control.

### DETAILED DISCRIPTION OF THE INVENTION

Unless otherwise defined, all technical and scientific terms used herein have the same meaning as commonly understood by those skilled in the art to which the present invention pertains. It should be understood that the terminology used herein is for the purpose of describing specific embodiments only, and is not intended to be limiting.

As used herein, the singular forms "a", "an" and "the" include plural references unless explicitly indicated otherwise. Thus, for example, reference to "a sample" includes a plurality of such samples and their equivalents known to those skilled in the art.

As used herein, the terms "around", "about" or "approximately" can generally mean within 20 percent, particularly within 10 percent, and more particularly within 5 percent of a given value or range. Numerical quantities given herein are approximate, meaning that the term "around", "about" or "approximately" can be inferred if not expressly indicated.

The term "edible carrier" as used herein refers to a carrier that is compatible with the other ingredients of the composition and is not deleterious to a subject. The compositions may contain other components, and may be formulated, for example, by employing conventional solid or liquid vehicles or diluents, as well as additives of a type appropriate to the mode of oral administration (for example, excipients, binders, preservatives, stabilizers, flavours, etc.) according to techniques such as those well known in the art.

According to the invention provides a composition for sleep and mood support, which comprises *Xylaria nigripes* extract, *Panax notoginseng* extract and *Cuscuta chinensis* extract at a relative ratio to provide a synergistic effect on GABA content, GABA-related enzyme activities, β-amyloid protein aggregation in PC12 cells.

In one embodiment of the invention, the composition for sleep and mood support consists essentially of *Xylaria nigripes* extract, *Panax notoginseng* extract and *Cuscuta chinensis* extract at a relative ratio to provide a synergistic effect on GABA content, GABA-related enzyme activities, β-amyloid protein aggregation in PC12 cells.

In one specific embodiment of the invention, the composition for sleep and mood support consists of *Xylaria nigripes* extract, *Panax notoginseng* extract and *Cuscuta chinensis* extract, as main active components, at a relative ratio to provide a synergistic effect on GABA content, GABA-related enzyme activities, β-amyloid protein aggregation in PC12 cells, and an edible carrier.

In one embodiment of the invention, the composition comprises/consists essentially of/consists of 50%-96% *Xylaria nigripes* extract, 0.001%-10% *Panax notoginseng* extract and 4%-50% *Cuscuta chinensis* extract.

In one preferred embodiment of the invention, the composition comprises/consists essentially of/consists of 70%-90% *Xylaria nigripes* extract, 0.01%-5% *Panax notoginseng* extract and 10%-30% *Cuscuta chinensis* extract.

In one more preferred embodiment of the invention, the composition comprises/consists essentially of/consists of 80%-90% *Xylaria nigripes* extract, 0.05%-1% *Panax notoginseng* extract and 10%-20% *Cuscuta chinensis* extract.

*Xylaria nigripes,* is an edible fungus (WulinShen) belonging to the Xylariaceae family. *Xylaria nigripes* has been traditionally used for treating various ailments including insomnia, anxiety, and neurodegenerative disorders (Zhou et al., 2022). It is commonly grown on dead trees and near abandoned termite nests (Hung et al., 2015). Recently many researchers have shown that *Xylaria nigripes* possesses various biological functions like antioxidant, anti-inflammatory, immunomodulatory, and anti-depressive properties along with hepato-protective and neuroprotective activities (Peng et al., 2015; Song et al., 2011; Ko et al., 2011). The above-mentioned activities are due to the presence of various phytocomponents including polyphenols, adenosine, triterpenoids, and polysaccharides (Divate et al., 2017; Ma et al., 2013). Ample amount of evidence indicated that *Xylaria nigripes* would improve GABA production and thus plays a key role in reducing depression and induce sleep (Zhoa et al., 2014; Wu et al., 2012). In the present invention, the *Xylaria nigripes* extract is obtained from the mycelium of *Xylaria nigripes,* particularly the fermented extract of *Xylaria nigripes* mycelium.

*Panax notoginseng* (root) is a popular traditional Chinese medicine (Chinese Ginseng) rich in ginsenosides (saponins). Previous studies have shown that ginsenosides from *Panax notoginseng* have considerably improved memory (cognitive function) and learning ability along with anti-depression, antioxidant and anti-inflammatory properties and thus altering the amyloid genesis process (Lin et al., 2019; Chen et al., 2019). Likewise, Zheng and his colleagues (2019) in his study confirmed that ginsenosides from Ginseng could enhance the production of acetylcholine and GABA as well as lower β-amyloid protein accumulation and thereby attenuates Aβ-amyloid protein-induced neuronal damage. In the present invention, the *Panax notoginseng* is obtained from the roots of *Xylaria nigripes,* particularly the water extract of *Panax notoginseng* roots.

*Cuscuta chinensis* is traditional medicine and is rich in flavonoids, phenolic acids, and polysaccharides (Donnapee et al., 2014). Previously, various scientists have proved that *Cuscuta chinensis* exhibits numerous pharmacological activities including antioxidant, anti-inflammatory, anti-depressant, anti-aging as well as renoprotective, hepatoprotective, and neuroprotective properties (Lin et al., 2018; Donnapee et al., 2014; Mokhtarifar et al., 2012). Zhen and others (2006), indicated that *Cuscuta chinensis* could considerably improve the survival rate of PC12 cells via lowering oxidative stress and thereby exhibiting neuroprotective properties. In the present invention, the *Panax notoginseng* is obtained from the seeds of *Xylaria nigripes,* particularly the ethanol extract of *Panax notoginseng* seeds.

According to the invention, Some examples of the composition for sleep and mood support are given below:

| | Xylaria nigripes extract | *Panax notoginseng* extract | Cuscuta Chinensis Extract |
|---|---|---|---|
| Feasible range | 50%-96% | 0.001%-10% | 4%-50% |
| preferred range | 70%-90% | 0.01%-5% | 10%-30% |
| More preferred range | 80%-90% | 0.05%-1% | 10%-20% |
| Composition 1 | 85% | 1% | 14% |
| Composition 2 | 80% | 0.1% | 19.9% |
| Composition 3 | 81.5% | 0.5% | 18% |
| Composition 4 | 88% | 0.05% | 11.95% |
| Composition 5 | 89.5% | 0.5% | 10% |
| Composition 6 | 75% | 1% | 24% |
| Composition 7 | 60% | 0.01% | 39.99% |

The present invention is further illustrated by the following examples, which are provided for the purpose of demonstration rather than limitation.

### Examples

### Methods

### Preparation of three compositions

Three highly purified botanical extracts including XE, XPE, and XPCE extracts were tested for the study. The three compositions were provided by NuLiv Sciences USA, inc. (Brea, CA, USA): XE *(Xylaria nigripes* extract) rich in polysaccharides and flavonoids, XPE *(Xylaria nigripes* extract and *Panax notoginseng* extract) rich in ginsenosides and XPCE *(Xylaria nigripes* extract, *Panax notoginseng* extract and *Cuscuta chinensis* extract) rich in polysaccharides and flavonoids.

The *Xylaria nigripes* extract was obtained from the mycelium of *Xylaria nigripes,* particularly the fermented *Xylaria nigripes* extract. The *Panax notoginseng* extract was obtained from the roots of *Panax notoginseng,* particularly the water extract of *Panax notoginseng* roots. The *Cuscuta chinensis* extract was obtained from the seeds of *Cuscuta chinensis,* particularly the extract of *Cuscuta chinensis* seeds with a 65%-95% ethanol solution.

### Culturing of PC12 cells

The PC12 cell line was purchased from BCRC (Hsinchu, Taiwan). PC12 cells were grown under Dulbecco's modified Eagle's medium (DMEM, Invitrogen) supplemented with 5% fetal bovine serum (FBS), 10% horse serum, and 50 µg/L gentamycin in a humidified atmosphere of 5% CO₂ at 37°C. Experiments were performed only when PC12 cells grown to ~80% confluence between passages 2-6. Before three compositions (XE, XPE and XPCE) treatments at different concentrations (40-120 µg/mL), the cells were grown in FBS/horse serum and an antibiotic-free medium for 16 h. The protein content of PC12 cell lysate was estimated using the BCA protein assay kit (ab102536) provided by Abcam (Cambridge, UK).

### GABA content

GABA contents in PC12 cells treated with different concentrations of the three compositions (XE, XPE and XPCE) were assayed by BioVision Quick Detect-GABA ELISA kit (E4457) purchased from BioVision (MA, USA) according to the manufacturer's protocol.

### GABA related enzymes

### GABA Transaminase (GABA-T) activity

GABA-T activity in PC12 cells was detected using a GABA-T assay kit (E-134) provided by Biomedical Research Service Center and Application (NY, USA) based on the supplier's instruction.

### GABA Decarboxylase (GAD) activity

GAD activity was assayed using BioVision Glutamate Decarboxylase Activity Assay Kit (K2021) purchased from BioVision (MA, USA) according to the manufacturer's protocol.

### PC12 cells viability with and without Aβ 1-42 peptide

PC12 cells were plated in 96 well plates at a density of 30,000 cells/well and incubated with each Zylaria composition at different concentrations (30-120 µg/mL) for 24 h at 37 °C. Add 10 µl of the CCK-8 solution (provided with a Cell counting kit) to each well of the plate. Incubate the plate for 24 h in the incubator and the absorbance at 450 nm was measured using a microplate reader. Also, the cell viability of PC12 cells with Aβ₁₋₄₂ (cytotoxic) peptide was also measured similarly as mentioned above to confirm the neuroprotective activity of the three compositions. In brief, Aβ₁₋₄₂ (10 µM) was diluted with serum-free culture medium and incubated with PC12 cells (30,000 cells/well) for 24 h and then treated with 3 different Zylaria compositions for another 24 h. Then, the PC12 cell viability was determined by using the Cell Counting Kit-8 (CCK-8) as mentioned above.

### Aβ protein Aggregation

The process of beta-amyloid protein aggregation was monitored using the ThT assay kit. ThT can assemble with misfolded amyloid proteins and emit fluorescence as a result of this union. Initially, Aβ₁₋₄₂ (10 µM final concentration) peptide/protein was mixed in equal volumes with each Zylaria composition (30-120 µg/mL), and ThT (200 µM final concentration) was added on 96-well plates. The ThT fluorescence was measured using a multi-detector microplate reader fluorescence spectrophotometer set at 450 nm for excitation and 485 nm for emission wavelengths. Fluorescence emission data were recorded every 5 minutes over a 3h period.

### Statistical analysis

All the experiments were performed in triplicate (n=3). Data are expressed as the mean ± standard deviation (SD). All statistical analysis was conducted using SPSS software, version 17 (SPSS, IBM, NY, USA). All the values were evaluated using one-way ANOVA, followed by Duncan's multi-comparison test to compare the difference between the three compositions at different concentrations. Differences were deemed significant when the probability values were less than 0.05 (p < 0.05).

### Results

### Evaluation of GABA content in PC12 cells after treatment with the three compositions

As shown in Figure 1 providing the GABA content (ng/ mg protein) and Table 1 listing the relative GABA content (%) in PC12 after treatment with the three compositions, the GABA content was considerably increased in PC12 cells, which were treated with the three compositions. Particularly, the 120 µg/mL concentration of XE (138.1%), XPE (139.4%), and XPCE (180.8%) showed the highest GABA levels than other concentrations. However, XPCE compositions rich in polysaccharides, flavonoids, and saponins exhibited the best GABA enhancing property (increase GABA synthesis) as compared to XE and XPE.

**Table 1. Impact of the three compositions on GABA content (%) in PC12 cells.**

| **Dose** (µg/mL) | **Relative GABA contents (%) in PC-12 cells** | | | |
|---|---|---|---|---|
| | **Control** | **XE** | **XPE** | **XPCE** |
| 20 | | 103.0±3.9 | 111.5±3.1* | 126.9±4.8* |
| 40 | | 110.5±.4.0 | 121.9±.4.* | 165.2±2.6*** |
| 60 | 100.0±3.2 | 120.0±2.9* | 128.5±3.9** | 171.4±3.4*** |
| 80 | | 122.2±4.7* | 132.9±4.6** | 174.2±6.6*** |
| 120 | | 138.1±4.3** | 139.4±7.5** | 180.8±7.9*** |

Data are expressed as the mean ± standard deviation (SD). *p < 0.05, **p < 0.01, ***p < 0.001 vs control.

### GABA Transaminase (GABA-T) activity in PC12 cells after treatment with the three compositions

The GABA-T activity in PC12 cells after treatment with the three compositions and control were shown in Table 2 (%) and Figure 2. The relative activity of GABA-T was considerably suppressed by all the three compositions (XE/XPE/XPCE) in PC12 cells in a dose-dependent manner than the control group. Here also, XPCE (120 µg/mL; 60.2%) displayed the highest (p < 0.001) GABA-T inhibitory activity and subsequently improved GABA content.

**Table 2. Impact of the three compositions on GABA Transaminase (GABA-T) activity (%) in PC12 cells.**

| **Dose** (µg/mL) | **GABA-T activity (%) in PC-12 cells** | | | |
|---|---|---|---|---|
| | **Control** | **XE** | **XPE** | **XPCE** |
| 40 | 100.0±4.4 | 87.5±.5.9 | 82.6±.10.3 | 71.8±.7.8* |
| 60 | | 86.6±4.0* | 68.8±8.8** | 73.9±.6.1* |
| 80 | | 79.5±6.0* | 69.7±13.2** | 67.2±8.7** |
| 120 | | 82.9±3.3* | 63.4±12.7** | 60.2±9.4*** |

Data are expressed as the mean ± standard deviation (SD). *p < 0.05, **p < 0.01, ***p < 0.001 vs control.

### GABA Decarboxylase (GAD) activity in PC12 cells after treatment with the three compositions

As shown in Table 3 (%) and Figure 3, the GAD activity in PC12 cells were significantly increased after treatment with XE (p < 0.05; p < 0.01), XPE (p < 0.01; p < 0.001), and XPCE (p < 0.001) with different concentration. XPCE displayed greater GAD enhancing activity in a dose-dependent fashion than XE and XPE.

**Table 3. Impact of the three compositions on GABA Decarboxylase (GAD) activity (%) in PC12 cells.**

| **Dose** (µg/mL) | **Relative GAD activity (%) in PC-12 cells** | | | |
|---|---|---|---|---|
| | **Control** | **XE** | **XPE** | **XPCE** |
| 40 | 100.0±5.3 | 105.6±3.5 | 121.9±5.7 | 136.3±2.6** |
| 60 | | 114.7±3.1 | 137.8±4.1** | 145.6±8.4*** |
| 80 | | 126.3±2.6* | 141.3±2.6** | 167.6±19.4*** |
| 120 | | 134.6±4.0** | 150.4±5.3*** | 220.8±12.3*** |

Data are expressed as the mean ± standard deviation (SD). *p < 0.05, **p < 0.01, ***p < 0.001 vs control.

### Cells viability with and without Aβ 1-42 peptide in PC12 cells after treatment with the three compositions

Initial analysis indicated the cell viability of PC12 cells was significantly increased when treated with the the three compositions for 24 h. The result showed that the three compositions had no cytotoxic activity against the PC-12 cell (Table 4 and Figure 4). XPCE composition exhibited a higher proliferative effect (p < 0.001) on the PC-12 cells than XE and XPE compositions.

**Table 4. Impact of the three compositions on Cells viability without Aβ 1-42 peptide (normal condition) exposure in PC12 cells.**

| **Dose** (µg/mL) | **Relative cell viability (%) under normal conditions in PC12 cells** | | | |
|---|---|---|---|---|
| | **Control** | **XE** | **XPE** | **XPCE** |
| 40 | 100.0±8.5 | 140.3±3.0* | 146.1±4.6** | 164.0±4.6*** |
| 60 | | 152.6±3.3** | 155.5±4.8** | 169.6±2.6*** |
| 80 | | 154.0±2.9** | 160.9±4.2*** | 174.5±4.7*** |
| 120 | | 157.7±4.8** | 165.1±3.6*** | 182.4±10.9*** |

Data are expressed as the mean ± standard deviation (SD). *p < 0.05, **p < 0.01, ***p < 0.001 vs control.

However, the cell viability of PC12 cells exposed to Aβ₁₋₄₂ peptides for 24 h, followed by pretreatment with the three compositions has shown a considerable increase in a dose-dependent manner (Table 5 and Figure 5). In addition, XPCE (120 µg/mL) displayed the highest proliferative and cytoprotective activity (p < 0.001) as compared to other compositions (XE and XPE).

**Table 5. Impact of the three compositions on Cells viability with Aβ 1-42 peptide exposure in PC12 cells.**

| **Dose** (µg/mL) | **Relative cell viability (%) under Aβ1-42 exposed condition in PC12 cells** | | | |
|---|---|---|---|---|
| | **Control** | **XE** | **XPE** | **XPCE** |
| 40 | 100.0±4.7 | 121.1±9.5 | 117.8±8.4 | 124.9±5.0* |
| 60 | | 130.7±12.3* | 130.2±15.2* | 140.5±9.5** |
| 80 | | 137.0±5.5** | 134.6±10.8** | 146.6±14.5** |
| 120 | | 139.5±11.9** | 143.3±15.9** | 157.3±13.1*** |

Data are expressed as the mean ± standard deviation (SD). *p < 0.05, **p < 0.01, ***p < 0.001 vs control.

### Aβ protein Aggregation in PC12 cells after treatment with the three compositions

The PC12 cells exposed with Aβ₁₋₄₂ peptides and followed by the addition of the three compositions showed potent inhibitory activity of beta-amyloid protein aggregation and subsequent neuronal injury/damage. All the the three compositions showed good anti-Aβ₁₋₄₂ peptides aggregation properties (Figure 6). Nevertheless, XPCE exhibited the highest (p < 0.001) inhibitory activity of beta-amyloid protein aggregation in a dose-dependent fashion as compared with XE and XPE compositions. Table 6 shows the percentage change in inhibition of Aβ protein aggregation (%). Based on the above data, the author confirmed that XPCE composition showed better neuroprotective activity than XE and XPE compositions.

**Table 6. Impact of the three compositions on inhibition of Aβ protein aggregation (%) in PC12 cells.**

| **Dose** (µg/mL) | **Inhibition of amyloid-beta aggregation (%) in PC12 cells** | | | |
|---|---|---|---|---|
| | **Control** | **XE** | **XPE** | **XPCE** |
| 40 | 100.0±8.3 | 88.4±1.0 | 70.3±11.9* | 67.2±8.1** |
| 60 | | 84.2±0.3* | 67.0±12.7** | 65.3±10.8** |
| 80 | | 83.4±1.0* | 65.4±7.4** | 65.1±7.5** |
| 120 | | 82.2±0.2* | 57.8±10.8*** | 56.9±5.8*** |

Data are expressed as the mean ± standard deviation (SD). *p < 0.05, **p < 0.01, ***p < 0.001 vs control.

### Discussion

It was the first time to conduct an experiment to check the combinations of different botanical compositions (XE, XPE and XPCE) on the GABA system and Aβ peptides aggregation. Since, various researchers have hinted that many Chinese traditional medicines like *Xylaria nigripes, Panax notoginseng,* and *Cuscuta chinensis* could positively maintain GABA levels and also lower Aβ peptides deposition in neuronal cells to exhibit potent neuroprotective activity (Lin et al., 2018; Donnapee et al., 2014; Song et al., 2011). Hence, this novel combination of botanical compositions (XE, XPE and XPCE) was used to check the holistic effect on GABA production and lower beta-amyloid protein production and its aggregation. GABA plays a major role in maintaining a healthy mental status, as it regulates various neurotransmitters/neurotropic factors and hormones production and thus useful in treating insomnia and depression (Yu et al., 2020; Solas et al., 2015) as well as various neuropsychological issues especially autism, schizophrenia, and other hyperactive neuronal disorders (Sarawagi et al., 2021; Sarasa et al., 2020).

GABA shunt system play a crucial role in maintaining proper balance in GABA levels (metabolism of GABA) using various GABA metabolizing enzymes including GAD and GABA-T (Sarawagi et al., 2021). In the present study, all the the three compositions treatments effectively improved the GABA production and hence the GABA content was considerably improved. The author hypothesizes that the rich content of flavonoids, saponins, and polysaccharides in the three compositions might influence the GABA synthesis. Previously, many studies have proved that (*Xylaria nigripes, Panax notoginseng, Cuscuta*) would trigger GABA production and thus plays a key role in reducing depression and insomnia (Forouzanfar et al., 2020; Zheng et al., 2018; Zhao et al., 2014; Wu et al., 2012). To confirm the above statement, the author would like the check the activity of two major enzymes like GAD and GABA-T, which are involved in the GABA shunt system. The activities of GAD and GABA-T were considerably improved and suppressed respectively by all three compositions (XE, XPE and XPCE) in PC12 cells in a dose-dependent manner. As the concentration of XE, XPE and XPCE increased the activity of GAD and GABA-T were significantly altered. However, the 120 µg/mL of XPCE composition showed the highest GABA synthesizing property (elevated GABA content), via increasing the activation of GAD and lowering the activity of GABA-T. Wu and other (2012), also indicated that *Xylaria nigripe* can increase the permeability of glutamate and thus enhance the activity of GAD, which in turn regulate the activity of GABA-T and thus maintain the nominal levels of GABA.

To cross-check the cytoprotective and proliferative activity the author decided to evaluate the PC12 Cell's viability with or without Aβ₁₋₄₂ peptide (cytotoxin). The PC12 cells after treatment with three compositions showed potent cytoprotective activity by increasing the PC12 cells count (proliferative activity) after 24 h of exposure to Aβ₁₋₄₂ peptide. The above statement proved that three compositions in particular XPCE displayed good cytoprotective and proliferative properties due to the presence of saponins and flavonoids. Likewise, Divate and his colleagues (2017), proved that the GABA-like property of Xylaria helps to protect the cells from H₂O₂-induced cytotoxicity. Also, ginsenosides (saponins) are reported to improve PC12 cell proliferation and could effectively prevent MMP-induced cytotoxicity (Hashimoto et al., 2012). As mentioned previously that Aβ peptide/protein is a neurotoxin, which is the major contributor to various neurodegenerative disorders especially Alzheimer's disease (AD) (Solas et al., 2015). To assess the neuroprotective property of three compositions, the PC12 cells were exposed to Aβ peptide/protein and the amount of Aβ peptide/protein aggregation/deposition was measured. The present data indicated that PC12 cells exposed to Aβ peptide/protein and followed by the addition of three compositions showed significant inhibitory activity of beta-amyloid protein aggregation/deposition. In view of the experiment results, the compositions of three botanical extracts (XPCE) exhibited a potent neuroprotective activity. In addition, the compositions of three botanical extracts (XPCE) showed the highest inhibitory activity of beta-amyloid protein aggregation than each of the three extracts or the combination of two botanical extracts. It was proved by the experimental data that the compositions of three botanical extracts (XPCE) had higher cytoprotective and neuroprotective properties than either *Xylaria nigripes* extract (XE) or a composition of *Xylaria nigripes* extract and *Cuscuta chinensis* extract (XCE), but lower oxidative stress (suppress free radical production) as well as inhibited Aβ protein misfolded/aggregation (altering various mitochondrial proteins), thus possibly lowering the onset of Alzheimer's Disease (AD) (Zheng et al., 2019; Shim et al., 2017; Choi et al., 2014).

### Conclusion

The three compositions (XE, XPE and XPCE) displayed good glutamate uptake and GABA release through elevated glutamate decarboxylase activity with decreased GABA transaminase activity, but the composition of three botanical extracts is the best. Also, the three compositions showed potent neuroprotective activity by improving the cell viability of PC12 cells and significantly lowering the Aβ aggregation. However, XPCE showed the best neuroprotective and GABA releasing properties than XE and XPE owing to high contents of various phytocomponents like polysaccharides, flavonoids, and saponins (holistic activity). The above data clearly showed that the composition of three botanical extracts exhibiting an improved effect on GABA levels and exhibiting strong neuroprotective activity, which makes these novel composition (XPCE) as a potent candidate for treating insomnia, depression and various neurological conditions (e.g., Alzheimer's Disease).

The invention has been described with reference to various specific and preferred embodiments and techniques. However, one skilled in the art will understand that many variations and modifications may be made while remaining within the spirit and scope of the invention

### References

1. Byun JI, Shin YY, Chung SE, Shin WC. Safety and efficacy of gamma-aminobutyric acid from fermented rice germ in patients with insomnia symptoms: a randomized, double-blind trial. Journal of clinical neurology. 2018 Jul;14(3):291-5.
2. Chen C, Zhang H, Xu H, Zheng Y, Wu T, Lian Y Ginsenoside Rb1 ameliorates cisplatin-induced learning and memory impairments. Journal of ginseng research. 2019 Oct 1;43(4):499-507.
3. Choi SM, Kim BC, Cho YH, Choi KH, Chang J, Park MS, Kim MK, Cho KH, Kim JK. Effects of flavonoid compounds on β-amyloid-peptide-induced neuronal death in cultured mouse cortical neurons. Chonnam medical journal. 2014 Aug;50(2):45-51.
4. Divate RD, Wang PM, Wang CC, Chou ST, Chang CT, Chung YC. Protective effect of medicinal fungus Xylaria nigripes mycelia extracts against hydrogen peroxide-induced apoptosis in PC12 cells. International Journal of Immunopathology and Pharmacology. 2017 Mar;30(1): 105-12.
5. Donnapee S, Li J, Yang X, Ge AH, Donkor PO, Gao XM, Chang YX. Cuscuta chinensis Lam.: A systematic review on ethnopharmacology, phytochemistry and pharmacology of an important traditional herbal medicine. Journal of ethnopharmacology. 2014 Nov 18;157:292-308.
6. Forouzanfar F, Vahedi MM, Aghaei A, Rakhshandeh H. Hydroalcoholic extract of Cuscuta epithymum enhances pentobarbitalinduced sleep: possible involvement of GABAergic system. Current Drug Discovery Technologies. 2020 Jun 1;17(3):332-7.
7. Hashimoto R, Yu J, Koizumi H, Ouchi Y, Okabe T. Ginsenoside Rb1 prevents MPP+-induced apoptosis in PC12 cells by stimulating estrogen receptors with consequent activation of ERK1/2, Akt and inhibition of SAPK/JNK, p38 MAPK. Evidence-Based Complementary and Alternative Medicine. 2012 Jan 1;2012.
8. Hung MC, Tsai CC, Hsu TH, Liang ZC, Lin FY, Chang SL, Ho WJ, Hsieh CW. Biological activities of the polysaccharides produced from different sources of Xylaria nigripes (Ascomycetes), a Chinese medicinal fungus. International Journal of Medicinal Mushrooms. 2015;17(2).
9. Kendell SF, Krystal JH, Sanacora G. GABA and glutamate systems as therapeutic targets in depression and mood disorders. Expert opinion on therapeutic targets. 2005 Feb 1;9(1): 153-68.
10. Ko HJ, Song A, Lai MN, Ng LT. Immunomodulatory properties of Xylaria nigripes in peritoneal macrophage cells of Balb/c mice. Journal of ethnopharmacology. 2011 Dec 8;138(3):762-8.
11. Lin J, Gao S, Wang T, Shen Y, Yang W, Li Y, Hu H. Ginsenoside Rb1 improves learning and memory ability through its anti-inflammatory effect in Aβ1-40 induced Alzheimer's disease of rats. American journal of translational research. 2019;11(5):2955.
12. Lin MK, Lee MS, Huang HC, Cheng TJ, Cheng YD, Wu CR. Cuscuta chinensis and C. campestris attenuate scopolamine-induced memory deficit and oxidative damage in mice. Molecules. 2018 Nov 22;23(12):3060.
13. Ma YP, Mao DB, Geng LJ, Zhang WY, Wang Z, Xu CP. Production optimization, molecular characterization and biological activities of exopolysaccharides from Xylaria nigripes. Chemical and Biochemical Engineering Quarterly. 2013 Jul 1;27(2):177-84.
14. Mokhtarifar N, Sharif B, Naderi N, Mosaddegh M, Faizi M. Evaluation of anti-depressant effects of Cuscuta chinensis in experimental models. Research in Pharmaceutical Sciences. 2012 Sep 2;7(5):826.
15. Ngo DH, Vo TS. An updated review on pharmaceutical properties of gamma-aminobutyric acid. Molecules. 2019 Jul 24;24(15):2678.
16. Peng WF, Wang X, Hong Z, Zhu GX, Li BM, Li Z, Ding MP, Geng Z, Jin Z, Miao L, Wu LW. The anti-depression effect of Xylaria nigripes in patients with epilepsy: A multicenter randomized double-blind study. Seizure. 2015 Jul 1;29:26-33.
17. Sarasa SB, Mahendran R, Muthusamy G, Thankappan B, Selta DR, Angayarkanni J. A brief review on the non-protein amino acid, gamma-amino butyric acid (GABA): its production and role in microbes. Current microbiology. 2020 Apr;77(4):534-44.
18. Sarawagi A, Soni ND, Patel AB. Glutamate and GABA homeostasis and neurometabolism in major depressive disorder. Frontiers in psychiatry. 2021 Apr 27;12:637863.
19. Shim JS, Song MY, Yim SV, Lee SE, Park KS. Global analysis of ginsenoside Rg1 protective effects in β-amyloid-treated neuronal cells. Journal of ginseng research. 2017 Oct 1;41(4):566-71.
20. Solas M, Puerta E, J Ramirez M. Treatment options in alzheimer s disease: the GABA story. Current pharmaceutical design. 2015 Oct 1;21(34):4960-71.
21. Song A, Ko HJ, Lai MN, Ng LT. Protective effects of Wu-Ling-Shen (Xylaria nigripes) on carbon tetrachloride-induced hepatotoxicity in mice. Immunopharmacology and Immunotoxicology. 2011 Sep 1;33(3):454-60.
22. Wu D, Xie ZX, Di P. Clinical application and efficacy of Wuling capsule. J China Prescript Drug. 2012;10:43-5.
23. Yu L, Han X, Cen S, Duan H, Feng S, Xue Y, Tian F, Zhao J, Zhang H, Zhai Q, Chen W. Beneficial effect of GABA-rich fermented milk on insomnia involving regulation of gut microbiota. Microbiological research. 2020 Mar 1;233:126409.
24. Zhao Z, Li Y, Chen H, Huang L, Zhao F, Yu Q, Xiang Z, Zhao Z. Xylaria nigripes mitigates spatial memory impairment induced by rapid eye movement sleep deprivation. International Journal of Clinical and Experimental Medicine. 2014;7(2):356.
25. Zhen GH, Jiang B, Bao YM, Li DX, An LJ. The protect effect of flavonoids from Cuscuta chinensis in PC12 cells from damage induced by H2O2. Zhong yao cai= Zhongyaocai= Journal of Chinese Medicinal Materials. 2006 Oct 1;29(10):1051-5.
26. Zheng M, Xin Y, Li Y, Xu F, Xi X, Guo H, Cui X, Cao H, Zhang X, Han C. Ginsenosides: a potential neuroprotective agent. BioMed Research International. 2018 May 8;2018.
27. Zheng Y, Gao X, Chen Q, Yan P, Liu R, Liang H, Zhang M. Effect of Ginsenosides on Prevention of Alzheimer's Disease. InIOP Conference Series: Materials Science and Engineering 2019 Oct 1 (Vol. 612, No. 2, p. 022004). IOP Publishing.
28. Zhou H, Zhao Y, Peng W, Han W, Wang D, Wang Z, Ren X, Pan G, Lin Q, Wang X. Efficacy and safety of Wuling capsule for insomnia disorder: A systematic review and meta-analysis of randomized controlled trials. Sleep Medicine. 2022 Mar 24.

## Claims

1. A composition for sleep and mood support, which consists essentially of *Xylaria nigripes* extract, *Panax notoginseng* extract and *Cuscuta chinensis* extract, as main active components at a relative ratio to provide a synergistic effect in increasing GABA content, and GABA-related enzyme activities, and decreasing β-amyloid protein aggregation.

2. The composition of claim 1, which consists of *Xylaria nigripes* extract, *Panax notoginseng* extract and *Cuscuta chinensis* extract, as main active components, at a relative ratio to provide a synergistic effect on GABA content, GABA-related enzyme activities, β-amyloid protein aggregation in PC12 cells, and optionally an edible carrier.

3. The composition of claim 1, wherein the *Xylaria nigripes* extract is obtained from the mycelium of *Xylaria nigripes* extract, the *Panax notoginseng* extract is obtained from the roots of *Panax notoginseng,* and the *Cuscuta chinensis* extract is obtained from the seeds of *Cuscuta chinensis.*

4. The composition of claim 2, which consists of 50%-96% *Xylaria nigripes* extract, 0.001%-10% *Panax notoginseng* extract and 4%-50% *Cuscuta chinensis* extract, and optionally an edible carrier.

5. The composition of claim 3, which consists of 70%-90% *Xylaria nigripes* extract, 0.01%-5% *Panax notoginseng* extract and 10%-30% *Cuscuta chinensis* extract, and optionally an edible carrier.

6. The composition of claim 4, which consists of 80%-90% *Xylaria nigripes* extract, 0.05%-1% *Panax notoginseng* extract and 10%-20% *Cuscuta chinensis* extract, and optionally an edible carrier.

7. The composition of claim 1, which provides cytoprotective and neuroprotective properties, but an efficacy in decreasing oxidative stress as well as inhibiting Aβ protein misfolded/aggregation.

8. The composition of claim 1, which is effective for treating insomnia, depression or a neurological condition.

9. The composition of claim 7, wherein the neurological condition comprises Alzheimer's Disease (AD).

10. A composition consisting essentially of *Xylaria nigripes* extract, *Panax notoginseng* extract and *Cuscuta chinensis* extract for use in treating insomnia, depression or a neurological condition.

11. The composition for use of claim 10, wherein the composition consists of *Xylaria nigripes* extract, *Panax notoginseng* extract and *Cuscuta chinensis* extract as main active components, at a relative ratio to provide a synergistic effect on GABA content, GABA-related enzyme activities, β-amyloid protein aggregation in PC12 cells, and optionally an edible carrier.

12. The composition for use of claim 10, wherein the neurological condition comprises Alzheimer's Disease (AD).
